# EUROPEAN PATENT SPECIFICATION

(11) **EP 4 441 507 B1**
(45) Date of publication and mention of the grant of the patent: **23.09.2026**
(21) Application number: 22822388.9
(22) Date of filing: 25.11.2022
(51) Int. Cl.: G01N 33/68

(54) **METHOD AND TOOLS FOR THE DETERMINATION OF CONFORMATIONS AND CONFORMATIONAL CHANGES OF PROTEINS AND OF DERIVATIVES THEREOF**
VERFAHREN UND WERKZEUGE ZUR BESTIMMUNG VON KONFORMATIONEN UND KONFORMATIONSÄNDERUNGEN VON PROTEINEN UND DEREN DERIVATE
PROCÉDÉ ET OUTILS DE DÉTERMINATION DE CONFORMATIONS ET DE MODIFICATIONS DE CONFORMATION DE PROTÉINES ET DE LEURS DÉRIVÉS

(30) Priority: 03.12.2021 EP 21212313
(43) Date of publication of application: 09.10.2024
(73) Proprietor: Biognosys AG, 8952 Schlieren (CH); ETH Zurich, 8092 Zürich (CH)
(72) Inventor: PICOTTI, Paola, 8049 Zürich (CH); VIZOVISEK, Matej, 8052 Zürich (CH); RINNER, Oliver, 8046 Zürich (CH); REITER, Lukas, 8800 Thalwil (CH); BEATON, Nigel, Framingham, MA 01702 (US); BRUDERER, Roland, 8406 Winterthur (CH); SABINO, Fabio Mira Rocha, 2850 Nærum (DK)
(74) Representative: Bremi, Tobias Hans
(86) International application number: PCT/EP2022/083223
(87) International publication number: WO 2023/099341

(56) References cited:
- WO-A1-2014/082733
- SCHOPPER S. ET AL.: "Measuring protein structural changes on a proteome-wide scale using limited proteolysis-coupled mass spectrometry", NATURE PROTOCOLS, vol. 12, no. 11, 26 October 2017 (2017-10-26), pages 2391 - 2410, XP055922516
- MA R. ET AL.: "Chemo-selection strategy for limited proteolysis experiments on the proteomic scale", ANAL. CHEM., vol. 90, no. 23, 7 November 2018 (2018-11-07), pages 14039 - 14047, XP055922666
- HEUSEL M. ET AL.: "Complex-centric proteome profiling by SEC-SWATH-MS", MOL. SYST. BIOL., vol. 15, no. 1, E8438, 14 January 2019 (2019-01-14), pages 1 - 22, XP055920805
- MINIC Z. ET AL.: "Chromatographic separation strategies for precision mass spectrometry to study protein-protein interactions and protein phosphorylation", J. CHROMATOGRAPHY B, vol. 1102, 24 October 2018 (2018-10-24), pages 96 - 108, XP085529364

## Description

### TECHNICAL FIELD

The present invention relates to methods for the determination of conformation and conformational changes of proteins and of derivatives thereof, optionally in their native biological context, in particular using limited proteolysis e.g. combined with selected reaction monitoring, data-dependent acquisition (DDA), data-independent acquisition (DIA), including Sequential Windowed Acquisition of All Theoretical Fragment Ion Mass Spectra (SWATH) methods and the like.

### PRIOR ART

Proteins are crucial effectors and regulators of a wide variety of cellular processes. In response to perturbations (for example, in case of disease), they can change their cellular concentration, activity, location and their structure. Being able to capture such transitions is an essential task in life sciences, to understand the functioning of basic cellular processes in health and disease and to identify new options for disease diagnosis and treatment. Changes in cellular protein concentration in response to perturbations can be routinely probed by mass spectrometry (MS) based-proteomic techniques. Much less is known about switches in cellular protein conformation, mostly due to the lack of suitable approaches to study protein folds in cells. This is a substantial limitation for biological and clinical applications, since conformational changes can strongly impact protein activity, location and stability, thus profoundly affecting a cell's physiology.

Proteins can change their conformation upon binding to lipids, ions, small molecules or nucleic acids, interaction with other proteins, chemical modification (e.g. phosphorylation) or environmental changes, such as varying pH, ionic strength or temperature. The extent of a conformational change ranges from small local motions, such as allosteric/local rearrangements, through larger scale fluctuations, such as domain motions, to the drastic switch between folded and unfolded or monomeric and polymeric states. In particular, the transition of monomeric proteins to higher order aggregated structures has gained increasing attention recently, in both biology and biomedicine. Over the last two decades, a variety of human diseases (more than 20 different pathologies), referred to as protein aggregation diseases were shown to be associated with the intracellular or extracellular accumulation of aggregates of specific misfolded proteins. Many neurodegenerative diseases, such as Parkinson's disease or Alzheimer's disease, of previously unknown etiology now fall into this category. The different diseases can even be classified according to the major protein components of their aggregates, which also distinguish their clinical manifestations. For example, αSynuclein (αSyn)-containing Lewy bodies are typical for most types of Parkinsonism (PD), while amyloid-β peptide inclusions are produced in Alzheimer's disease (see. e.g. A Aguzzi & T O'Connor, Nat Rev Drug Discov 9 (3), 237). The possibility of monitoring such protein conformational transitions in biological specimens would open new possibilities for the diagnosis and therapy of these protein-centric conditions and shed light on their pathogenesis.

A number of biophysical techniques have been applied to monitor conformational features of proteins, such as nuclear magnetic resonance (NMR), X-ray crystallography, infrared and Raman spectroscopy, circular dichroism, atomic force microscopy or fluorescence spectroscopy. These techniques are predominantly used to analyze (purified) proteins in vitro, due to their incapability of dealing with complex biological backgrounds. This is a substantial limitation, since the conformation adopted by a protein is regulated in cells by multiple co-occurring events specific to its cellular context, such as environmental cues, binding events or post translational modifications, which cannot be recapitulated by in vitro systems. Techniques based on Förster resonance energy transfer (FRET) offer the advantage of monitoring conformational changes of proteins in their native cellular environment, but require the introduction of fluorescent probes at suitable sites of each target protein and are not applicable on a large scale or on clinical samples.

In light of the above considerations, the availability of methods for tracing protein conformational changes in their biological environment and in a multiplexed manner (multiple proteins at a time) is an urgent requirement. Additional features of an ideal method are: i) suitability for scale-up (fast analysis of multiple samples) and ii) uncomplicated adaptability to different applications (clinical or biotechnological applications or basic research in biology).

Gupta, Lapadula and Abou-Donia report in a paper entitled "Purification and Characterization of Cytochrome P450 Isozymes from β-Naphthoflavone-Induced Adult Hen Liver" (Archives of Biochemistry and Biophysics, 282 (1) 170-182 (1990)) on the purification and characterization of pure cytochrome P450. Characterization takes place by protease treatment using chymotrypsin under denaturing conditions.

Cohen, Ferré-D'Amaré, Burley and Chait propose in a paper entitled "Probing the solution structure of the DNA-binding protein Max by a combination of proteolysis and mass spectrometry" (Protein Science (1995), 4:1088-1099) a simple biochemical method that combines enzymatic proteolysis and matrix-assisted laser desorption ionization mass spectrometry to probe the solution structure of DNA-binding proteins. The method is based on inferring structural information from determinations of protection against enzymatic proteolysis, as governed by solvent accessibility and protein flexibility. WO-A-2014082733 discloses a limited proteolysis (LiP) protocol, i.e. a method for the detection of the conformational state of a protein contained in a complex mixture of further proteins and/or other biomolecules, in particular in a complex native biological matrix, as well as assays for such a method. The method comprises, if needed after an extraction and/or lysis step, the following steps: 1. Limited proteolysis of the complex mixture under a condition where the protein is in the conformational state to be detected leading to a first fragment sample; 2. Denaturation of the first fragment sample to a denaturated first fragment sample; 3. Complete fragmentation of the denaturated first fragment sample in a digestion step to a completely fragmented sample; 4. Analytical analysis of the completely fragmented sample for the determination of fragments characteristic of having been the result both the limited proteolysis of step 1. as well as of the complete fragmentation in the digestion step 3. for the determination of the conformational state.

Schopper et al report in a paper entitled "Measuring protein structural changes on a proteome-wide scale using limited proteolysis-coupled mass spectrometry" (nature protocols, VOL.12 NO.11, 2017, 2391ff) on protein structural changes induced by external perturbations or internal cues which profoundly influence protein activity and thus modulate cellular physiology. Limited proteolysis-coupled mass spectrometry (LiP-MS) is reported to be a recently developed proteomics approach that enables the identification of protein structural changes directly in their complex biological context on a proteome-wide scale. After perturbations of interest, proteome extracts are subjected to a double-protease digestion step with a nonspecific protease applied under native conditions, followed by complete digestion with the sequence-specific protease trypsin under denaturing conditions. This sequential treatment generates structure-specific peptides amenable to bottom-up MS analysis. Next, a proteomics workflow involving shotgun or targeted MS and label-free quantification is applied to measure structure-dependent proteolytic patterns directly in the proteome extract. Possible applications of LiP-MS include discovery of perturbation-induced protein structural alterations, identification of drug targets, detection of disease-associated protein structural states, and analysis of protein aggregates directly in biological samples. The approach also enables identification of the specific protein regions involved in the structural transition or affected by the binding event.

Jafari et al report in a paper entitled " The cellular thermal shift assay for evaluating drug target interactions in cells" (nature protocols, VOL.9, NO.9, 2014, 2101ff) thermal shift assays used to study thermal stabilization of proteins upon ligand binding. Such assays have been used on purified proteins in the drug discovery industry and in academia to detect interactions. A proof-of-principle study was published describing the implementation of thermal shift assays in a cellular format, which they call the cellular thermal shift assay (CETSA). The method allows studies of target engagement of drug candidates in a cellular context, exemplified with experimental data on the human kinases p38a and ERK1/2. The assay involves treatment of cells with a compound of interest, heating to denature and precipitate proteins, cell lysis, and the separation of cell debris and aggregates from the soluble protein fraction. Whereas unbound proteins denature and precipitate at elevated temperatures, ligand-bound proteins remain in solution. They describe two procedures for detecting the stabilized protein in the soluble fraction of the samples. One approach involves sample workup and detection using quantitative western blotting, whereas the second is performed directly in solution and relies on the induced proximity of two target-directed antibodies upon binding to soluble protein. The latter protocol has been optimized to allow an increased throughput, as potential applications require large numbers of samples.

Cappelletti et al report in a paper entitled "Dynamic 3D proteomes reveal protein functional alterations at high resolution in situ" (Cell 184, 545-559, January 21, 2021) that a global protein structural readout can be based on limited proteolysis-mass spectrometry (LiP-MS) which detects many functional alterations, simultaneously and in situ, in bacteria undergoing nutrient adaptation and in yeast responding to acute stress. The structural readout, visualized as structural barcodes, captured enzyme activity changes, phosphorylation, protein aggregation, and complex formation, with the resolution of individual regulated functional sites such as binding and active sites. Comparison with prior knowledge, including other 'omics data, showed that LiP-MS detects many known functional alterations within well-studied pathways. It suggested distinct metabolite-protein interactions and enabled identification of a fructose-1,6-bisphosphate-based regulatory mechanism of glucose uptake in E. coli. The structural readout dramatically increases classical proteomics coverage, generates mechanistic hypotheses, and paves the way for in situ structural systems biology.

Savitzki et al report in a paper entitled " Tracking cancer drugs in living cells by thermal profiling of the proteome" (sciencemag.org, 3 October 2014, VOL 346 ISSUE 6205) on performing thermal proteome profiling (TPP) on human K562 cells by heating intact cells or cell extracts and observed marked differences in melting properties between the two settings, with a trend toward increased protein stability in cell extract. Thermal profiling of cellular proteomes is reported to enable the differential assessment of protein ligand binding and other protein modifications, providing an unbiased measure of drug target occupancy for multiple targets and facilitating the identification of markers for drug efficacy and toxicity. Ma R. et al. : "Chemo-selection strategy for limited proteolysis experiments on the proteomic scale", Anal. Chem., vol. 90, no. 23, 7 November 2018, pages 14039-14047 describes a chemo-selective enrichment strategy, termed the semitryptic peptide enrichment strategy for proteolysis procedures (STEPP), to isolate the semitryptic peptides generated in mass spectrometry-based proteome-wide applications of limited proteolysis methods. The strategy involves reacting the ε-amino groups of lysine side chains and any N-termini created in the limited proteolysis reaction with isobaric mass tags. A subsequent digestion of the sample with trypsin and the chemo-selective reaction of the newly exposed N-termini of the tryptic peptides with N-hydroxysuccinimide (NHS)-activated agarose resin removes the tryptic peptides from solution, leaving only the semitryptic peptides with one nontryptic cleavage site generated in the limited proteolysis reaction for subsequent LC-MS/MS analysis. As part of this work, the STEPP technique is interfaced with two different proteolysis methods, including the pulse proteolysis (PP) and limited proteolysis (LiP) methods. The STEPP-PP workflow is evaluated in two proof-of-principle experiments involving the proteins in a yeast cell lysate and two well-studied drugs, cyclosporin A and geldanamycin. The STEPP-LiP workflow is evaluated in a proof-of-principle experiment involving the proteins in two cell culture models of human breast cancer, MCF-7 and MCF-10A cell lines. The STEPP protocol increased the number of semitryptic peptides detected in the LiP and PP experiments by 5- to 10-fold. The STEPP protocol not only increases the proteomic coverage, but also increases the amount of structural information that can be gleaned from limited proteolysis experiments. Moreover, the protocol also enables the quantitative determination of ligand binding affinities.

Heusel M. et al. : "Complex-centric proteome profiling by SEC-SWATHMS", Mol. Syst. Biol., vol. 15, no. 1, Article no. e8438, 14 January 2019, pages 1-22 describe an integrated experimental and computational technique to quantify hundreds of protein complexes in a single operation. The method consists of size exclusion chromatography (SEC) to fractionate native protein complexes, SWATH/DIA mass spectrometry to precisely quantify the proteins in each SEC fraction, and the computational framework CCprofiler to detect and quantify protein complexes by error-controlled, complex-centric analysis using prior information from generic protein interaction maps. The analysis of the HEK293 cell line proteome delineates 462 complexes composed of 2,127 protein subunits. The technique identifies novel sub-complexes and assembly intermediates of central regulatory complexes while assessing the quantitative subunit distribution across them. They make the toolset CCprofiler freely accessible and provide a web platform, SECexplorer, for custom exploration of the HEK293 proteome modularity.

Minic et al. "Chromatographic separation strategies for precision mass spectrometry to study protein-protein interactions and protein phosphorylation" J. CHROMATOGRAPHY B, vol. 1102, pages 96-108 report investigating protein-protein interactions and protein phosphorylation can be of great significance when studying biological processes and human diseases at the molecular level. However, sample complexity, presence of low abundance proteins, and dynamic nature of the proteins often impede in achieving sufficient analytical depth in proteomics research. In this regard, chromatographic separation methodologies have played a vital role in the identification and quantification of proteins in complex sample mixtures. The combination of peptide and protein fractionation techniques with advanced high-performance mass spectrometry has allowed the researchers to successfully study the protein-protein interactions and protein phosphorylation. Several new fractionation strategies for large scale analysis of proteins and peptides have been developed to study protein-protein interactions and protein phosphorylation. These emerging chromatography methodologies have enabled the identification of several hundred protein complexes and even thousands of phosphorylation sites in a single study. In this review, they focus on current workflow strategies and chromatographic tools, highlighting their advantages and disadvantages, and examining their associated challenges and future potential.

### SUMMARY OF THE INVENTION

Current LiP-MS approaches (e.g. Schopper et al or Cappelletti et al) as e.g. disclosed in WO2014082733 rely specifically on a double digestion workflow wherein a broadly unspecific enzymatic digest on native proteins is followed by a full tryptic digestion of the denatured proteome. The ensuing analysis is then focused on tryptic and/or semi-tryptic peptides. The approach proposed here, on the other hand, is designed to focus on the peptides that are largely not present in a typical LiP-MS experiment as they are generated only from native, non-denatured proteins and are largely non-tryptic. It is important to note that even if one were to attempt to utilize fully non-tryptic peptides in the analysis of a standard LiP-MS experiment this yields very little useful information for two reasons. First, very few fully non-tryptic peptides remain as a result of the complete tryptic digest done under denatured conditions that is standard in the LiP-MS protocol. Second, any such peptides that are generated are difficult to detect and quantify accurately via mass spectrometry as their signal is weak and/or masked by the sheer amount and volume of tryptic and/or semi-tryptic peptides present. Analysis of a typical LiP-MS experiment finds that <1% of total peptides are fully non-tryptic and do not contribute to target identification in positive control experiments.

The proposed workflow is a technique that enables MS-based identification of unique structural/conformational states of proteins and/or structural/conformational protein changes in complex biological or clinical specimens with high sensitivity, coverage and throughput using LC-MS, DIA (data-independent acquisition of product ion spectra) mass spectrometry and unspecific database searches.

The structural/conformational states of proteins sampled by the proposed approach can be natural, non-natural or a mixture of both, depending on the application. Implementations of this technique can be used to investigate protein structure under standard conditions, during protein binding to a drug/small molecule or metabolite, upon binding to other proteins (protein-protein interactions, i.e. protein complexes), upon binding to a variety of other molecules (e.g. lipids or DNA) as a result of a chemical modifications (e.g. PTMs, like protein phosphorylation), or a change of local environment (e.g. temperature increase, ionic strength changes or presence of chaotropes). The proposed technique allows the detection and identification of proteins that undergo structural changes in a hypothesis-free manner when a perturbation is induced in the investigated system (e.g. triggers of immune signaling or disease). Identifying such unique protein conformational changes and states provides valuable information about protein structure and function, enables the identification of targets of drugs or metabolites of interest, characterizes biochemical and signaling pathways involved in the response to the perturbation and informs on disease mechanisms. Furthermore, altered protein structures can be used as a proxy for disease detection (i.e. structural biomarkers). The insights into the structural state and dynamics of the structural proteome provide a deeper understanding of both physiological and non-physiological mechanisms of action, both of which can represent major hurdles in advancing our understanding of diseases, as well as supporting drug design and refinement. Thus, the proposed technique enables exploiting structural proteomics for a variety of applications ranging from basic biology, to target deconvolution and biomarker discovery.

More specifically, the proposed technique uses a novel approach to address problems/shortcomings that are inherent to existing mass spectrometric techniques that aim at addressing the aforementioned questions (e.g. the above mentioned approaches by Schopper et al, Cappelletti et al, Jafari et al and Savitzki et al). These techniques focus on maximizing the depth (coverage, sensitivity) of proteome discovery by relying on generation and identification of tryptic and/or semi-tryptic digested mixtures (i.e. one tryptic cleavage per peptide). These peptides are typically better suited for mass spectrometric analysis with respect to their size and identifiability. However, in these experiments many peptides are non-informative with respect to structural/conformational information while substantially adding to the complexity, dynamic range and noise of the sample. Furthermore, in the current version of LiP-MS, after tryptic digestion, many peptides will be too short for reliable MS-based identification and are thus lost in the analysis. The proposed technique is a new variant of the LiP-MS approach that instead focuses on an increased relative ability to identify peptides that convey structural/conformational information from proteins at the expense of peptide (and thus protein) identifications that do not necessarily report structural information. By focusing the attention on enrichment of structurally informative peptides, the proposed technique increases the signal-to-noise ratio, thus making the identification of protein structural changes more robust.

The key feature of the proposed technique is that it increases the number and abundance of truly informative peptides relative to the total number of peptides that are contained in a sample, thus strongly reducing the dynamic range challenge inherent to proteomics samples.

This problem is very pronounced for human body fluids such as blood plasma but also exists in samples where the protein(s) of interest are of low abundance such as is often the case for the study of drugs with single (or few) protein targets. Beside other factors, this broad dynamic range originates from the protein size/concentration distribution in combination with the number and distribution of peptide responses in the mass spectrometer. In a classical proteomic approach, including the LiP-MS approach, the larger a protein is, the more tryptic peptides it will generate. Hence there is a strong correlation between protein size and/or abundance, and the likelihood that such a protein will generate at least some peptides that have a strong response in the mass spectrometer. In contrast, the proposed technique approach produces peptides mainly from protease accessible regions of proteins that are in their native or near native conformation (i.e. not denatured). These peptides tend to be on the solvent-exposed surfaces of proteins. This substantially reduces the proportional number of peptides derived from large proteins since the relationship between protein surface area and volume is not a fixed ratio and on average decreases as a protein increases in size. By exploiting this surface area to size ratio bias the proposed technique aims at reducing the dynamic range inherent to proteomics samples that is due, at least in part, to the natural size distribution of proteins. In addition, the proposed technique focuses on the information rich accessible peptides that report on protein structure and protein structural changes.

The proposed technique exploits two key processes, namely a limited digestion coupled with enrichment of short, MS-compatible peptides. The collective intention of the procedure is to increase the number of peptides that convey conformational/structural information (signal), while decreasing the amount of peptides that do not contain such information (noise) in a mass spectrometer ready sample.

This is accomplished in a surprisingly simple and efficient approach by using a limited digestion step under non-denaturing (i.e. protein structure retaining) conditions, using a specific or non-specific protease. Limited digestion can be varied by modifying the enzyme to substrate ratio, performing the digest at lower temperature or by performing the digest on a relatively short time scale. The resulting peptide mixture is then, in contrast to the LiP-MS protocol, not fully denaturated and completely fragmented, but it is filtered or treated in other ways (see below) to remove large peptides/protein fragments, that represent a large fraction of the mixture and contain little information on the structure/conformation and/or structure/conformational changes of proteins. By removing large peptides/protein pieces from the digest prior to mass spectrometry, the proposed technique also reduces the number of non-informative peptides that could introduce artifacts and/or noise to the experiment from the perspective of peptide identification in the mass spectrometer and also during downstream data analysis. This removal can be achieved by a process that separates large peptides/protein pieces, to enrich for suitable peptides including size filtering (e.g. using a 10k MWCO filtration device), chromatography (e.g. size-exclusion, hydrophobic or anion exchange) or physical processes (e.g. phase separation, absorption or precipitation).

Compared to classical LiP-MS, the proposed technique omits a full trypsinization step under denaturing conditions after the limited proteolysis step.

In addition to a single protease being used for the limited digestion step, the proposed technique can be augmented by the use of protease mixtures and/or sensitizers (e.g. heat, urea). Instead of protease mixtures two distinct proteases (or sets of proteases) can also be used on an aliquot of the sample and the sample pooled afterwards. Pooling can be done after digestion or separation of peptides from the rest of the sample. Instead of pooling, the samples can also be processed and measured completely separately. Protease mixtures and sensitizers act in two ways to improve identification of proteins of interest according to the proposed technique. Protease mixtures include proteases that target different amino acids for cleavage and thus quite simply enable both more and unique peptides to be generated during the digestion step. Sensitizers work by slightly disrupting the native state of the protein to enable novel protease cleavages (cleavage sites). Sensitizers are particularly useful for the proposed technique when specific states are being investigated for changes in protein structure (e.g. with and without a drug or metabolite). In such cases the effect of sensitizers is magnified as proteins of interest will become more or less susceptible to the particular sensitizer if their structure has been changed (i.e. stabilized or destabilized) by an event such as binding of a drug.

In these ways, the proposed technique is able to identify specific protein conformations or structures by exploiting traditionally ignored peptides (i.e. peptides with two non-tryptic termini located at the surface of proteins). The step that removes large peptides and proteins (e.g. filtration) included in the proposed technique workflow although altering overall peptide/protein identification numbers, leads to a relative enrichment for structurally informative peptides.

The proposed technique workflow comprises or consists of the following steps:
1. A sample containing a protein or a proteome from cells, tissues, or body fluids is investigated for protein structure and/or conformational states. This includes but is not limited to incubation with a ligand (e.g. small molecule, metabolite, etc.) at defined concentrations (treatment mixture), including control (vehicle) samples or subjecting the lysate to conditions that induce a structural state change such as temperature, metabolic stimulant, etc. including unstimulated samples. Whatever conditions are utilized, native protein structures (primary, secondary, tertiary and preferably / potentially also quaternary structure) are preserved throughout.
2. Each sample is subjected to a limited digestion step using a specific or an unspecific protease (or a combination thereof). This digest should be relatively short, typically 1-5 minutes, and rapidly quenched.
3. Following this digestion step, the larger peptides and protein fragments are removed e.g. via filtration, separation or enrichment devices (see methods above and below).
4. The remaining peptides are then processed using a standard proteomics workflow (e.g. denaturation, C18 clean-up, etc) and analyzed via LC-MS/MS so that the peptides can be identified and quantified.

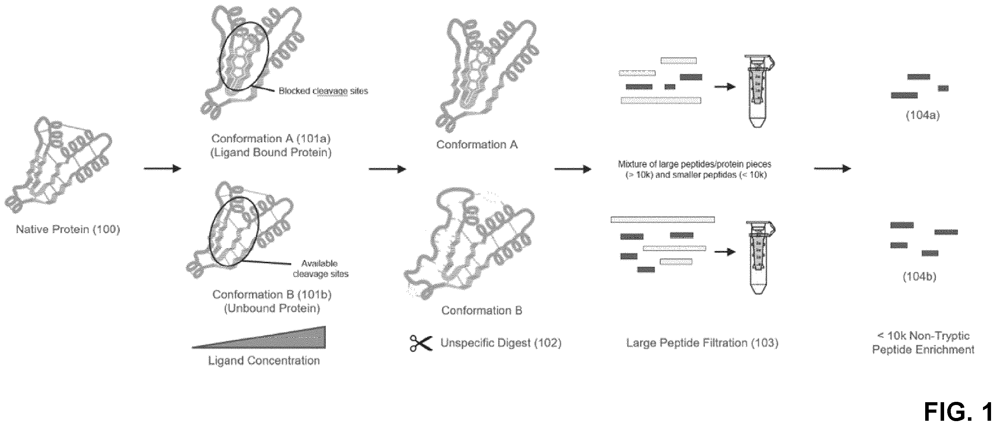

A schematic with an example of how the technique supports the detection of structural/conformational changes is shown in **Fig. 1****,** using the investigation of a ligand binding event as an example.

This way, the proposed approach is a novel complementary approach to LiP-MS that can provide entirely unique information from a traditional LiP-MS experiment because it focuses on generating and analyzing a unique set of peptides. By digesting only native proteins for a limited time, followed by the introduction of a filtration/enrichment step, the proposed technique reduces the number of information-poor peptides and/or protein pieces that are problematic during sample preparation, data acquisition and analysis. This boost in the signal-to-noise ratio can represent a significant improvement in data quality and subsequently in biological insights.

More generally speaking, the present invention relates to a method as claimed in claim 1 for the detection of the conformational state of at least one protein, said at least one protein being contained in a complex mixture of further proteins and/or other biomolecules (such a complex mixture can e.g. be a complex cell extract mixture, or generally a biological probe such as body fluids (plasma, cerebrospinal, urine,...), based on tissue, an environmental sample (e.g. sea water,...), biological secretions, e.g. obtained by cells lysed according to a published LiP protocol, see above, and protein concentration can be determined using an assay kit). Said at least one protein in said complex (e.g. cell extract) mixture has been subjected to a condition inducing a structural change in said at least one protein. The method comprises, if needed after an extraction and/or lysis step, the following sequence of steps:
1. Limited proteolysis of the complex (e.g. cell extract) mixture under a native condition in which the at least one protein is in the original conformational state to be detected leading to a first fragment sample; directly followed by
2. Removal of large peptides and proteins or other biomolecules from said first fragment sample to form an enriched fragment sample;
3. Analytical analysis of the enriched fragment sample for the determination of fragments characteristic of having been the result of the limited proteolysis of step 1. as well as remaining after the removal step 2. for the determination of the conformational state of said at least one protein.

When mentioning "directly followed" at the end of step 1., this excludes further denaturation and/or proteolysis steps but does not exclude further steps involved in the termination of the limited proteolysis of step 1., i.e. steps of quenching the limited proteolysis by adding corresponding reagents (e.g. Deoxycholic acid sodium solutions), increasing temperature, washing, filtering, sedimentation, solvent, ionic strength and/or pH adjustments, or a combination thereof and the like. In other words, during the limited proteolysis digestion, there are no other steps that contribute to the peptide generation (e.g. denaturation to increase cleavage site access or addition of other proteases, etc) so step 1. from a digestion perspective is 'complete'. However, prior to step 2 there can be an addition of deoxycholate and bringing the temperature up to e.g. 98°C, which is done to stop the protease activity from step 1.

The terminology "conformational state" of at least one protein is to be understood broadly as commonly accepted in the field, meaning the arrangement in space of the protein's constituent atoms determining the overall shape of the molecule. In other words the expression "conformational state" includes any kind of information going beyond the information of the primary structure, i.e. the linear sequence of amino acids and potential chemical modifications thereof in a peptide or protein. The expression "conformational state" therefore includes the secondary structure (three dimensional arrangement of local segments of proteins, the two most common secondary structural elements are alpha helices and beta sheets, but this also includes beta turns and omega loops), the supersecondary structure (motives, compact three-dimensional protein structure of several adjacent elements of a secondary structure that is smaller than a protein domain or a subunit), the tertiary structure (domains, three dimensional shape of the protein) and the quaternary structure (structure of proteins which are themselves composed of two or more smaller protein chains, also referred to as subunits).

Conformational changes in proteins, which can be identified using the proposed method, are made possible by their intrinsic flexibility. These changes may occur with only relatively small expenditure of energy. At the molecular structural level, conformational changes in single polypeptides are the result of changes in main chain torsional angles and side chain orientations. The overall effect of such changes may be localised with reorientations of a few residues and small torsional changes in the regional main chain. On the other hand torsional changes localised at very few critically placed residues may lead to large changes in tertiary structure. The later type of conformational changes is described as domain motions. Tobi et al, report in a paper entitled " Structural changes involved in protein binding correlate with intrinsic motions of proteins in the unbound state" (PNAS December 27, 2005vol. 102 no. 52) that the conformational changes associated with protein-protein interactions vary from local changes in side chains rotameric states to global changes in structure such as collective domain movements.

The proposed approach is a structural proteomics approach, wherein structural proteomics is defined here as using a proteomics approach to determine structural features/structures of proteins and structural changes of proteins for individual proteins or proteome wide. The proposed method also enables what is termed target deconvolution. Target deconvolution is the identification of direct interaction partners of a drug/small molecule or protein. Target deconvolution can be achieved by numerous methods including; affinity chromatography, expression-cloning, protein microarray, 'reverse transfected' cell microarray, and biochemical suppression.

The analysis of step 3 is carried out by quantitative mass spectrometry.

The proposed method is based on the coupling of a biochemical technique called limited proteolysis (LiP) and an advanced targeted mass spectrometry workflow, involving DIA (including SWATH-MS) or other mass spectrometry approaches such as selected reaction monitoring (SRM) or SRM-like approaches including Parallel Reaction Monitoring (PRM), Data-Dependent Acquisition (DDA), isobaric labelling quantification.

Liquid chromatography coupled to Mass Spectrometry (LC-MS) has been used for many years in the proteomic community for the identification and quantification of peptides (and thus proteins) from complex sample mixtures. The commonly most used approaches are variants of the so called LC-MS/MS or "shotgun" MS approach that is based on the generation of fragment ions from precursor ions that are automatically selected based on the precursor ion profiles (data dependent analysis, DDA). The most mature technology is called selected Reaction Monitoring (SRM), frequently also referred to as multiple reaction monitoring (MRM). The targets for MRM experiments are defined on a rational basis and depend on the hypothesis to be tested in the experiment. Selected combinations of precursor ions and fragment ions (so called transitions, the set of transitions for one target precursor is called MRM assays) for these targets are programmed into a mass spectrometer, which then generates measurement data only for the defined targets. Another variant of targeted proteomics is data independent acquisition, and a more recently presented variant commonly called SWATH-MS approach or Data Independent Acquisition (DIA). Here, the targeted aspect is introduced only on the data analysis level. Contrary to MRM, this approach does not require any preliminary peptide specific method design prior to the sample injection. Since the LC-MS acquisition covers the complete analyte contents of a sample through the entire mass and retention time (RT) ranges the data can be mined a posteriori for any peptide/precursor of interest. Data is acquired in a data independent manner, on the complete mass range (e.g. 200-2000 Thomson) and through the entire chromatography, disregarding of the content of the sample. This is commonly achieved by stepping the peptide precursor selection window step by step through the complete mass range. In effect, this data acquisition method generates a complete fragment ion map for all the analytes present in the sample and relates the fragment ion spectra back to the precursor ion selection window in which the fragment ion spectra were acquired. This is achieved by widening the precursor isolation windows and thus accounting a priori for multiple precursors co-eluting and concomitantly participating to the fragmentation pattern recorded during the analysis. Such a precursor window is called a swath. The result is complex fragment ion spectra from multiple precursor fragmentations, that require a more challenging data analysis. Unlike in shotgun proteomics, for the MRM and SWATH or DIA technology spectra are repeatedly recorded for the same analytes with a high time resolution (LC retention time resolution). The (high) time resolution when compared to shotgun proteomics, together with the limited fragment ion information for MRM and the limited fragment ion to precursor ion association for SWATH/DIA, makes a completely new type of data analysis necessary and possible. Since only a limited number of pre-defined analytes are being monitored, it is not necessary to make a shotgun proteomics type database search by comparing the spectra to a complete theoretical proteome. Instead, a number of scores have been described that are based on signal features such as shape, co-elution of transitions, and similarity of transition intensities to assay libraries. The completely new type of data analysis are the targeted (peptide centric) and untargeted data (spectrum centric) analysis.

Spectrum-centric analysis can be defined as follows: data analysis of data obtained in an LC-MS/MS experiment, which can be DDA or DIA data, in which the search is spectrum centric. This means that the spectra in the MS2 dimension are scanned for possible matches with all theoretical peptides and their fragments derived from a protein database typically with no or limited prior spectral information. Typically, the parent precursor ion for a MS2 spectrum is matched with a certain m/z tolerance to the theoretical m/z for all precursors in the search space giving a set of candidate peptides . Then the candidate peptide which best explains the spectra in terms of theoretical fragment ions is considered as the peptide spectrum match (PSM). No further prior information on the fragments is required.

Peptide-centric analysis/peptide centric search can be defined as follows: data analysis of data obtained in an LC-MS/MS experiment, which can be DDA or DIA data, in which the search is precursor centric. The predicted possible peptides and their fragments derived from a predicted spectral library or an empirical spectral library are queried against the spectra in the MS1 and MS2 dimension . In this analysis, spectral information of the peptides is required, in particular retention time, ion mobility, and likely to be observed fragment ions with relative fragment intensities. This information is used to narrow the search space of the peptide by querying only the spectrum that falls within a certain m/z, iRT or IM tolerance and for scoring of matches. Having this additional information greatly improves the sensitivity of the analysis by leading to more powerful scores.

Furthermore, confidence estimation of identification in MRM by means of false discovery rates cannot be done as for the classical shotgun proteomics. Therefore, a novel approach has been developed by measuring transitions for non-existing peptides (decoy transitions) (Reiter L, Rinner O, Picotti P, Huttenhain R, Beck M, Brusniak MY, Hengartner MO, Aebersold R: mProphet: automated data processing and statistical validation for large-scale SRM experiments. Nature methods 2011, 8(5):430-435). The data from these decoy transitions can be used to derive false discovery rates as is done in shotgun proteomics. This confidence estimation by means of false discovery rate is necessary to determine the data significance level and allow user defined quality filtering of the data. SWATH/DIA data are distinct from MRM data. In contrast to MRM, full fragment ion spectra are recorded using the SWATH/DIA method. The time resolution is usually chosen similarly as in MRM. When comparing SWATH/DIA with shotgun proteomics, the difference is that in SWATH/DIA the fragment ion spectra are derived from a much higher number of precursors because the window for precursor selection is usually chosen as wide (e.g. up to or as high as 25Th or up to 32Th instead of roughly 1Th for shotgun proteomics. This high complexity of the fragment ion spectra makes it unpractical/inefficient to analyze the data as in shotgun proteomics using database searches. However, the data can be analyzed similarly to MRM data with the additional benefit of the high time resolution in the data. This can be done by extracting ion currents corresponding to transitions in MRM. The resulting data can then be analyzed very similarly to MRM. In all variants of LC coupled mass spectrometry, proteins in samples for MRM experiments are digested into smaller peptides prior to the analysis. The resulting peptide mixture is usually chromatographically separated in order to reduce the complexity of the sample. Chromatographic separation adds a time dimension to the recorded data of the mass spectrometer, the retention time (RT). Data independent acquisition data can also be analyzed in a spectrum-centric or untargeted fashion where queries are made in the search space based on the data, for instance based on the precursor ion (MS1) signals in the data (unlike for SWATH). This is the same analysis type that is typically used for DDA. Further, various quantification technologies can be used such as isobaric labelling such as TMT or iTRAQ or stable isotope labeling with amino acids in cell culture (SILAC) or isotopically heavy labelled peptides can be added for the absolute quantification of peptides and proteins. Also parallel reaction monitoring (PRM) can be used which is similar to MRM but it is performed on a high resolution instrument and fragment ion scans (MS2 scans) are acquired in full range for the analyte(s) that are targeted in the analysis.

SRM assays are specific, quantitative mass spectrometry-based assays for peptides or proteins of interest, akin to antibodies for Western blotting, but with higher multiplexing capabilities and lower development time (assays for 100 peptides can be developed in one hour). We previously demonstrated that SRM allows quantifying proteins in a broad range of cellular abundances, down to <50 copies per cell, in total cell lysates (see P Picotti et al., Cell 138 (4), 795 (2009); and Picotti at al. Nature Methods, VOL.9 NO.6, JUNE 2012), resolving proteins with high (>95%) sequence overlap and measuring target peptides across large numbers of samples. Therefore, this technology enables quantitative measurements of specific peptides in very complex samples. Recently, further developments of the SRM approach include SRM-like approaches based on data-independent acquisition of product ion spectra and their targeted analysis (SWATH method, see LC Gillet et al., Mol Cell Proteomics 11 (6), O111 016717 (2012).

According to a first preferred embodiment, for the detection of the conformational state as such in parallel to steps 1.-3. the original complex mixture (cell extract) with said at least one protein and without being subjected to said condition inducing a structural change is subjected to steps 1. - 3. for the generation of an enriched fragment control sample, and wherein the determination of the conformational state of the at least one protein is based on a quantitative comparison of the analytical analysis of the enriched fragment sample with the analytical analysis of the enriched fragment control sample.

According to another preferred embodiment, for the detection of a change of the conformational state depending on different conditions in the complex mixture, a first and a second complex mixture is generated by subjecting them to the different conditions inducing a structural change in said at least one protein, by individually subjecting the two complex mixtures to steps 1.-3., and wherein the determination of the conformational change of the at least one protein is based on a comparison of the analytical analysis of the first enriched fragment sample with the analytical analysis of the second enriched fragment sample. Typically, the condition inducing a structural change in said at least one protein in said complex (cell extract) mixture is preferably selected from the group consisting of: temperature change; pressure change; ionic strength change; pH change; metabolic stimulant change; ligand addition, including drug/small molecule addition, metabolite addition, protein addition, peptide addition, lipid addition, DNA addition, RNA addition, disease/health state or status and genetic variations (e.g. mutations, etc), or a combination thereof; addition of a chaotrope; chemical modification, including post translational modifications, in particular phosphorylations, disulphide bridge formation, ADP-ribosylation, ubiquitination, SUMOylation, acetylation, methylation, oxidation, glycosylation, or a combination thereof.

Preferably, in step 2. peptides and proteins are removed in a filtration, separation or any other enrichment step.

For the purpose of the invention, the term "enrichment" can be defined as follows: The limited proteolysis of the complex (cell extract) mixture under a condition in which at least one protein is in the original conformational state to be detected leads to a first fragment sample. The removal e.g. via filtration, separation or enrichment devices of large peptides and proteins or other biomolecules not of interest from said first fragment sample corresponds to the enrichment step leading to an enriched fragment sample.

Preferred methods are including size filtering (for example using a 10 k MWCO filtration device); chromatography including size exclusion, hydrophobic or anion exchange chromatography; physical removal including phase separation, absorption, precipitation; filtration, separation or enrichment based on hydrophilic/hydrophobic properties; filtration, separation or enrichment based on electric/magnetic field; or a combination thereof.

Filtration can also be performed with two or more filters such that a specific peptide size range is enriched, i.e. not only removing large peptides but also very small ones that will not be specific enough because of a very short amino acid sequence or that are not suited for mass spectrometric analysis.

In step 2. peptides, proteins or other biomolecules or both having a molar weight larger than 20 kDa, preferably having a molar weight larger than 15 kDa, most preferably having a molar weight larger than 10 kDa are removed from the first fragment sample.

In line with the above good results can also be obtained if, as preferred, in step 2. Also peptides, having a molar weight smaller than 0.1 kDa, or having a molar weight smaller than 0.2 kDa, or having a molar weight smaller than 0.4 kDa are removed from the first fragment sample.

According to another preferred embodiment, step 3. includes, before actual analysis, a proteomics workflow, in particular involving denaturation, C18 cleanup, or a combination thereof.

According to a preferred protocol, in the limited proteolysis step 1. a proteolytic system selected from the group consisting of protease K, Thermolysin, Subtilisin, Pepsin, Papain, α-Chymotrypsin, Elastase, and mixtures thereof is used.

In step 1. the proteolytic system is preferably used at a concentration, with respect to the total biomolecular content in the sample, given as the ratio of enzyme to biomolecular content, in the range of 1/50-1/10000, preferably in the range of 1/100-1/1000 by weight. Step 1. can be carried out over a time span of 1-60 minutes, preferably in the range of 2-30 minutes, or 2 - 10 minutes or 2 - 5 minutes, further preferably at a temperature in the range of 20-40°C.

Preferably the temperature in the limited proteolysis step 1 is in the range of 20-40°C or 4-90°C. The temperature range is normally at around room temperature (20-25°C) or at 37°C; thermolysin on the other hand is active up to 80°C; 4 °C also applicable to slow down proteolytic reaction.

The properties of the used unspecific proteases are summarized in the table below:

| *Protease* | *Optimal pH* | *Specificity* |
|---|---|---|
| Proteinase K | 7.5 - 11.0 | Unspecific |
| Thermolysin | 7.0- 9.0 | Leu, Phe,Ile, Val, Met, Ala |
| Subtilisin | 7.0 - 11.0 | Unspecific |
| Pepsin | 1.0 - 4.0 | Unspecific |
| Papain | 6.0 - 7.0 | Unspecific |
| α-Chymotrypsin | 7.0 - 9.0 | Phe, Tyr, Trp, Leu, Ile |
| Elastase | 7.5 - 8.8 | Ala, Val, Ile, Leu, Gly, Ser, Thr |

For quantitative determination heavy labelled fragments characteristic of being the result of the limited proteolysis of step 1. as well as remaining after the removal step 2., can be spiked into the original complex mixture and/or into the first fragment sample and/or into the enriched fragment sample. So if desired, absolute quantitation can be achieved using heavy-labelled synthetic internal standard peptides. The approach can be directly applied to unfractionated proteome extracts, or it can be coupled to a variety of isotope-labeling and sample fractionation techniques (for example to iTRAQ and TMT labeling and the TAILS workflow, O Kleifeld et al., Nature biotechnology 28 (3), 281 (2010)), previously used in proteomic experiments.

For the analytical analysis in step 3., preferably specific, quantitative mass spectrometry-based assays in the form of selected reaction monitoring (SRM) and/or data-independent acquisition of product ion spectra (DIA) are used.

Normally, the complex (e.g. cell extract) mixture of further proteins and/or other biomolecules is a complex native biological matrix.

The at least one protein is normally a protein based exclusively on proteinogenic amino acids, or is based on proteinogenic amino acids and carries post-translational modifications. Furthermore the present invention relates to the use of a method as detailed above for the determination of a medically relevant conformation of the protein, for the determination of protein-based drugs, for the influence of drugs or other ligands on proteins, or for quality control of protein-based pharmaceutical preparations.

Also the present invention relates to the use of a method as detailed above in combination with peptide fragment enrichment techniques such as TAILS for the peptides generated by the step 1.

The proposed method opens numerous possibilities in biomedical, biotechnological and pharmaceutical applications as well as in basic and biological research, only some of them shall be given. It provides a novel platform to measure protein conformational changes, additionally to the conventional protein abundance changes or protein modification changes, currently measured by MS.
- This has particular potential for the detection and treatment (testing new drugs) of diseases caused by protein misfolding and aggregation, such as Alzheimer's or Parkinson's diseases. Conformotypic peptides can be used to probe the structure of disease-related proteins in clinical samples and have potential as disease biomarkers. Furthermore they can be used to test the capability of chemical modulators (drugs) to influence the aggregation process directly in cell extracts, in drug screens.
- This technique can also be applied to monitor stability and proper protein folding of protein-based drugs, an important quality control step for pharmaceutical companies in the production of drugs.
- Since proteins can change conformation upon binding of drugs or other ligands, the method can also be used to identify drug or ligand receptors, based on the detected conformational change.
- It can be used to probe the structure of protein receptors of interest, directly in their cellular matrix, thus aiding the design of molecules to target them.
- The marker assays can be translated into kits for the diagnosis of human diseases (disease biomarkers).
- The method can be used in the drug development pipeline, for quality control of protein-based pharmaceutical preparations.
- It can be used for the development of new drugs for protein conformational diseases.
- It can aid in the identification of receptors of existing drugs, to understand their mechanism of action.

Further embodiments of the invention are laid down in the dependent claims.

The invention is defined by the appended claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic of the proposed approach; native proteins are incubated with a ligand at defined concentrations, including control (vehicle) samples; each sample is subjected to a brief rapidly quenched limited digest step; next, larger peptides and protein pieces are removed), yielding unique peptide populations dependent upon protein conformation during the limited digest; a standard proteomics workflow can be implemented using the remaining peptides;
- Fig. 2: illustrates a comparison of the LiP-MS protocol with two different variants of the proposed Dark-LiP methodology using the drug Rapamycin;
- Fig. 3: illustrates the identification of peptides from FKBP1 by LiP-MS and compared with Dark-LiP;
- Fig. 4: illustrates a LiP-MS protocol comparison with two different variants of the Dark-LiP methodology using the general kinase inhibitor staurosporine;
- Fig. 5: illustrates a comparison of the LiP-MS protocol with the Dark-LiP protocol for identification of staurosporine protein targets, wherein A shows the total number of proteins and peptides identified by LiP-MS and Dark-LiP;, B shows the distribution of peptide length in LiP-MS and Dark-LiP, and C shows a graph illustrating the number of kinases identified as drug-target candidates (true positives) in function of the total number of drug-target candidates (true positives + false positives);
- Fig. 6: describes an experimental design for the identification of protein structural differences with Dark-LiP;
- Fig. 7: illustrates a comparative analysis of limited proteolysis of TAU monomer and TAU fibril.

### DESCRIPTION OF PREFERRED EMBODIMENTS

Fig. 1. shows a schematic of the proposed approach where the differentiating condition for the conformational difference between the reference and the altered sample is the addition of a ligand. Native proteins 100 are incubated in the altered sample with a ligand 101a at defined concentrations (upper path), while no ligand is added in the control (vehicle) samples 101b (lower path). Each sample is subjected to a brief (1-5 minutes), rapidly quenched limited digest step 102, typically with an unspecific protease. Next, larger peptides and protein pieces are removed (e.g. via filtration) in step 103, yielding unique peptide populations 104a/b that are dependent upon protein conformation during the limited digest 102. A standard proteomics workflow can be implemented thereafter using the remaining peptides (e.g. denaturation, C18 clean-up, LC-MS and analysis).

### Examples

### Example 1: comparison LiP-MS to proposed, so called Dark-LiP technologies

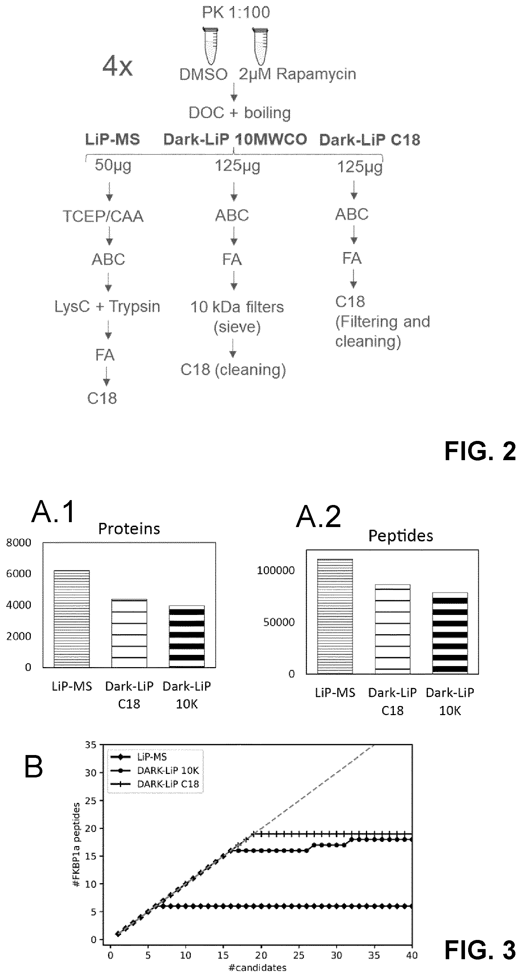

The results of Example 1 are illustrated in **Fig. 2** illustrating a comparison of the LiP-MS protocol with two different variants of the proposed approach, the Dark-LiP methodology using the drug Rapamycin. (FA: Formic acid, TCEP: reducing agent tris (2-carboxyethyl) phosphine, CAA: alkylating agent chloroacetamide, DOC: Deoxycholate, ABC: Ammonium bicarbonate, LysC: Endoproteinase LysC).

To compare the current LiP-MS protocol with the Dark-LiP setup, four aliquots of 300µg HeLa lysate were incubated with 2µM Rapamycin and four aliquots with the carrier (DMSO).Next, each aliquot was treated with Proteinase-K at a protein ratio of 1:100 (m/m). After stopping the reaction with sodium deoxycholate (DOC) and boiling, samples were divided in three aliquots.

One aliquot is used to evaluate LiP-MS downstream processing strategies.

Two aliquots are used to evaluate Dark-LiP downstream processing strategies.

For the LiP-MS approach, 50µg of protein extract was reduced, alchylated, diluted with ammonium bicarbonate buffer and digested with LysC and trypsin. Finally, the generated peptides were acidified with formic acid and cleaned with a C18 resin.

For Dark-LiP methodology, PK-digested cellular proteomes were diluted with ammonium bicarbonate and acidified with formic acid, thereby omitting sample reduction, alchylation and digestion with LysC/Trypsin.

The first variant of Dark-LiP consisted in, directly after the limited proteolysis, filtering the acidified sample through 10MWCO cut off spin filters before peptide cleanup with C18. For a second variant of Dark-LiP, directly after the limited proteolysis, the filtration step was omitted, and the PK-generated peptides were directly acidified and cleaned with a C18 resin.

For both the LiP-MS and Dark-LiP methodologies after C18 cleanup, the obtained purified peptides were analysed by mass spectrometry.

Identification of peptides from FKBP1 by LiP-MS and Dark-LiP.

**Fig. 3** shows the identification of peptides from FKBP1 by LiP-MS and Dark-LiP.

**Fig. 3A.1** shows the total number of *proteins* identified by LiP-MS and Dark-LiP. The two variants of Dark-LiP identified comparable number of proteins. LiP-MS identified the highest number of proteins.

**Fig. 3A.2** shows the total number of peptides identified by LiP-MS and Dark-LiP. The two variants of Dark-LiP identified comparable number of peptides. LiP-MS identified the highest number of peptides.

The first variant of Dark-LiP (consisting in, directly after the limited proteolysis, filtering the acidified sample through 10MWCO cut off spin filters before peptide cleanup with C18) is identified on **Fig. 3A.1** and **Fig. 3A.2** and **Fig. 3B** as 'Dark-Lip 10 K'.

The second variant of Dark-LiP (consisting in, omitting the filtration step directly after the limited proteolysis, the PK-generated peptides being directly acidified and cleaned with a C18 resin) is identified on **Fig. 3A.1** and **Fig. 3A.2** and **Fig. 3B** as 'Dark-Lip C18'.

**Fig. 3B** shows the number of FKBP1 peptides identified as drug-target candidates (true positives) in function of the total number of drug-target candidates (true positives + false positives).

The analysis by LiP-MS identified a higher number of peptides and proteins than Dark-LiP **(****Fig. 3****),** since the samples are inherently more complex without filtering of the large proteins and peptides. Since peptides are obtained after the fragmentation of proteins, it is expected that the removal of the fraction of large proteins in Dark-LiP also leads to a lower number of peptide identifications than in LiP-MS.

The number of proteins and peptides identified by the two variants of Dark-LiP are comparable, as well as the number of FKBP1 peptides (true positives). Consequently, we choose the "simpler" Dark-LiP variant for further experiments: the variant that omits the 10MWCO cut off spin filters.

To test the performance of Dark-LiP and LiP-MS on the identification of FKBP proteins (targets of Rapamycin) as drug-target candidates, we used the machine learning pipeline LiP-quant to score the peptides identified in the several analysis, obtaining a list of peptide drug-target candidates ranked by the LiP score.

The number of peptides derived from known, described Rapamycin targets (FKBP proteins) were plotted against the total number of peptide candidates (true positives + false positives) **(****Fig. 3****).** Hence, steeper lines mean a higher number of FKBP derived peptides than flatter line. The results show that both variants of the Dark-LiP methodology outperformed the standard LiP-MS by identifying more FKBP peptides with high score than LiP-MS.

LiP-Quant is a drug target deconvolution data analysis pipeline based on limited proteolysis coupled with mass spectrometry that works across species, including in human cells. Machine learning is used to discern features indicative of drug binding and integrate them into a single score to identify protein targets of small molecules and approximate their binding sites.

### Example 2: Comparison of the LiP-MS protocol with one variant of the Dark-LiP methodology (C18 variant) using the general kinase inhibitor staurosporine

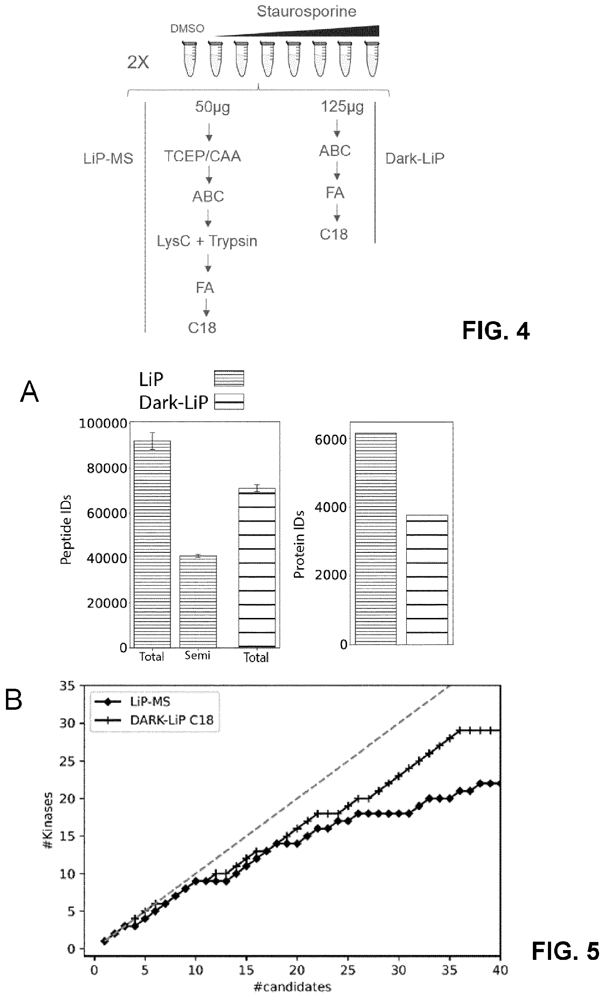

**Fig. 4** shows the comparison of the LiP-MS protocol with one variant of the DARK-LiP methodology (C18 variant) using the drug Staurosporine. (FA; Formic acid, TCEP: reducing agent, CAA: alkylating agent, DOC: Deoxycholate, ABC: Ammonium bicarbonate, LysC: Endoproteinase LysC).

We compared the performance of LiP-MS with Dark-LiP in a drug-dose response experimental setup, using the general kinase inhibitor staurosporine as model system **(****Fig. 4****).**

Kinases are a large family of enzymes that phosphorylate other proteins and are essential for normal cellular function. Due to the large number of kinases, the staurosporine assay is commonly used to compare the efficiency of different methodologies for drug-target identification.

175 µg of native HeLa protein lysates were treated with seven different concentrations of the drug staurosporine and DMSO in duplicates, and processed the samples accordingly to the LiP-MS protocol or the C18 variant of the Dark-LiP methodology, as described above.

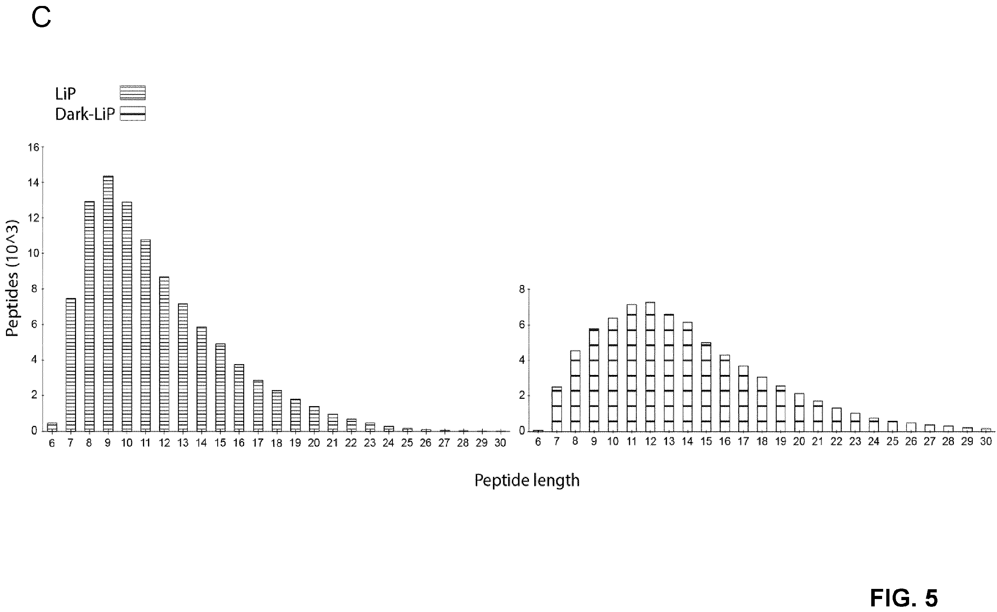

**Fig. 5** shows the comparison of LiP-MS with Dark-LiP for identification of staurosporine protein targets.

**Fig. 5A** shows the total number of proteins and peptides identified by LiP-MS and Dark-LiP.

**Fig. 5B** shows the distribution of peptide length in LiP-MS and Dark-LiP.

**Fig. 5C** shows the number of kinases identified as drug-target candidates (true positives) in function of the total number of drug-target candidates (true positives + false positives).

Comparably to the Rapamycin experiment, the analysis by LiP-MS identified a higher number of peptides and proteins than Dark-LiP, while Dark-LiP even identified more semi-tryptic peptides than LiP-MS **(****Fig. 5A****).** Moreover, the peptides identified by Dark-LiP were also larger in length, due to the absence of the second fragmentation step with Trypsin/LysC **(****Fig. 5B****).**

We used LiP-quant to score the identified peptides based on the correlation between peptide abundance and the drug concentration, thereby generating a list of the most probable staurosporine targets. To visually compare the performance of Dark-LiP and LiP-MS for the identification kinases as drug-targets, we plotted the number of kinases identified as target candidates against the total number of candidates **(****Fig. 5C****).** As described above, steeper lines mean a higher number of kinases identified as top drug-target candidates, representing a more efficient method. The results show that Dark-LiP performed better than LiP-MS by identifying more kinases as targets of staurosporine.

### Example 3: Dark -Lip technology allowing the detection of protein conformational changes.

It is estimated that more than 45 million people suffer from dementia worldwide, being Alzheimer's disease the most common form of dementia. Alzheimer's disease is characterized by the accumulation of abnormal fibrillar tangles of the microtubule-associated protein tau, a natively unfolded protein which harbors a highly flexible conformation under physiological conditions.

References exemplify clinically relevant conformational changes of a Tau protein in health and disease that can be discriminated by Dark-LiP.

In the Dark-LiP procedure during the limited-proteolysis step, proteinase-K will fragment the TAU proteins in solution. The speed of fragmentation of a particular region of TAU is dependent on the accessibility of that region to proteinase-K. Monomer TAU is described in the literature as a highly disordered protein, composed by protein segments with high flexibility. Thereby, the regions of monomer TAU are highly accessible to proteinase-K, which translates into a fast kinetics of fragmentation, and ultimately high abundant peptides. On the contrary, fibrillar TAU is described in the literature as a large structure of aggregated molecules of monomer TAU. This means that several regions and molecules of fibrillar TAU will be protected from proteinase-K by other regions and molecules of TAU that aggregated together. Overall, this lower accessibility translates into a slower speed of fragmentation, and consequently peptides with lower abundance.

To demonstrate this, three aliquots of 100µL LiP-MS buffer were used to dilute 6µg of monomer TAU, and three aliquots of 100µL LiP-MS were used to dilute 6µg of fibrillar TAU. Next, each aliquot was treated with Proteinase-K at a molecular ratio of 1:50 (50 molecules of TAU for each molecule of PK) and incubated for 2min at room temperature. The reaction was stopped by addition of sodium deoxycholate (DOC) and boiling.

Next, PK-fragmented TAU samples were diluted with ammonium bicarbonate and acidified with formic acid (thereby omitting sample reduction, alkylation and digestion with LysC/Trypsin, as characteristic from the Dark-LiP methodology). After acidification, PK-generated peptides were cleaned with a C18 resin. This step removes the large peptides and proteins, which are kept in the C18 resin.

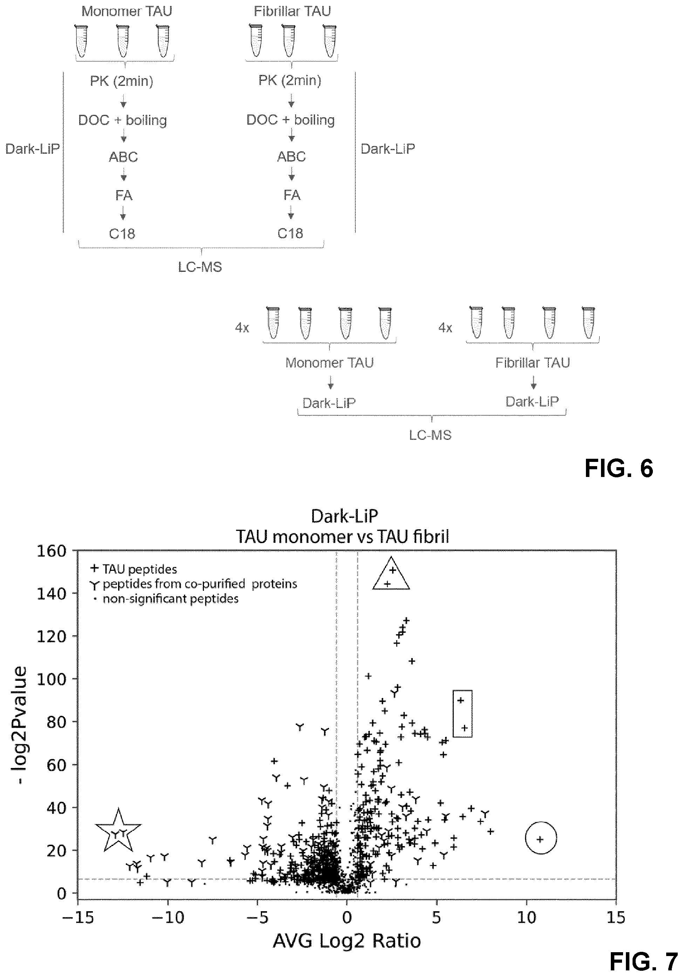

**Fig. 6** describes an experimental design for the identification of protein structural differences with Dark-LiP).

The fragmented TAU samples generated upon processing with the Dark-LiP workflow are analyzed by mass spectrometry, and the peptides identified in both conditions are compared using a statistical t-student test.

The Student's t-test for two samples is used to test whether two sample groups (two populations) are different in terms of a quantitative variable, based on the comparison of two samples drawn from these two groups (equation 1). In other words, a two-sample Student's t-test allows to test the null hypothesis whether the means of two populations are equal (with the samples being measured on a quantitative continuous variable). In the case of the Student's t-test, the mean and the standard error of the mean is used to compare the two samples and a normal distribution of the data is assumed.

The comparison of the means is performed accordingly to equation 1, and the output is the value *t*. This *t*-value is a measurement of the magnitude of the difference between the means of the two populations, in relation to the variability of measurements. The larger the value of *t*, the larger and more significant is the difference between the two populations, and the lower the variability among measurements. A particular *t*-value can then be transformed into the probability of obtaining that *t*-value (p-value) via the two-sample t distribution values. The relation between t-values and p-values was already established for a particular distribution of probabilities for a defined test, and therefore the p-value can be calculated directly (in this case we assume two-sample t-test and a normal distribution). The p-value (p stands for probability) is frequently used to measure statistical significance, and describes the likelihood of the observed differences in means) being explained by chance. P-values represent a probability from 0% to 100%, thus an example p-value of 0.01 correspond to a probability of 1%. Hence, the lower the p-value, the lower the probability of the observations being explained by chance, and consequently the higher statistical significance (in the described example, the observation would happen by chance in 1% of a random population of measurements). $t = \frac{\left({x}_{1}-{x}_{2}\right)}{\sqrt{\frac{{\left({s}_{1}\right)}^{2}}{{n}_{1}} + \frac{{\left({s}_{2}\right)}^{2}}{{n}_{2}}}}$
t : Measure of the size of difference between population, relative to the variability of measurements. t-values vary between 0 (no difference in means) and infinity.
x₁ : Mean of the measurements of population 1.
x₂ : Mean of the measurements of population 2.
s₁ : Standard deviation of the measurements of population 1.
s₂ : Standard deviation of the measurements of population 2.
n₁ : Number of measurements of population 1.
n₂ : Number of measurements of population 2.

In our example with the TAU protein, we use the t-test to calculate if the difference in abundance of a particular peptide generated by Dark-LiP fragmentation of monomer TAU and fibrillar TAU is statistically significant. If the difference in mean abundance between the three measurements performed for monomer TAU is different than the mean abundance of the three measurements performed for fibrillar TAU, we consider that the speed of fragmentation was different, and consequently the accessibility of that particular peptide to proteinase-K was also different between the two isoforms of TAU. Hence, peptides with statistically significant difference in abundance directly translate into structural difference of TAU. For global visualization of the results of the statistical analysis, we plotted the inverse of the logarithmic of the p-values (derived from the t-values obtained in equation 1) against the logarithmic of the fold change of peptide abundance for each peptide (ratio between x₁ and x₂ described in equation 1) **(****Fig. 7****).** Since p-values can vary highly between peptides, we use the logarithmic transformation to improve the visualization, otherwise the peptides with the lowest p-values would localize too far from the peptides with the highest p-values, and therefore would not be possible to visualize the data efficiently. We use the inverse of the logarithmic to transform the data into positive values which are easier to visualize and interpret, since the logarithmic of numbers lower than 1 are negative. By applying the logarithmic transformation to the fold changes, we can also efficiently visualize fold changes that vary highly in value, and discriminate between peptides when x1 is larger than x₂ (positive AVG Log2 Ratio), from peptides when x₁ is lower than x₂ (negative AVG Log2 Ratio).

Moreover, from equation 1 we observe that the p-values depend on the difference of abundance between the two populations, but also on the number of measurements and on the standard deviation of those measurements (which correlates to the variability of the observation between replicates). Hence, it is also important to understand if a p-value is mostly derived from a large difference of the means of the sample groups (fold change, obtained by the numerator of equation 1, x₁-x₂), or if the p-value derives mostly from the low standard error of the mean (low variability among measurements, denominator of equation 1, ((s₁/n₁)² + (s₂/n₂)²), since n₁=n₂= 3 replicates. Thereby, we plotted the p-values against the fold changes (ratio between x₁ and x₂), simultaneously visualizing the difference in means of the two sample groups (fold change), and an estimation of the variability of the measurements.

In proteomics studies, the value of 1% for significance and fold change of 2 are generally accepted by the community. Hence, peptides with a p-value larger than 0.01 (equal to - log2Pvalue of 6.64, represented by the horizontal dashed line) and with a fold change lower than 2 (log2Ratio lower than -1 and higher than 1, represented by the vertical dashed lines) were considered as statistically non-significant. These non-significant peptides are represented by small dots and localized in the areas below and between the dashed lines. Peptides with p-values lower than 0.01, and with a fold change higher than 2 were considered statistically significant. The statistically significant peptides derived from TAU are represented as cross shapes, while the "Y" shapes represent statistically significant peptides derived from bacterial proteins co-purified during the preparation of the TAU proteins. When x₁ is larger than x₂, the fold change (ration between x₁ and x₂) is larger than1, and the peptide shows in the right part of the graph. When x₂ is larger than x₁, the fold change is lower than 1, and the peptides show in the left part of the graph. Overall, peptides with high values in the y-axis correspond to peptides with low p-value, and consequently high statistical significance (which can be correlated with low standard deviations, and consequently high reproducibility among replicates). Symbols with high modular values in the x-axis correspond to high difference in abundance, which can be correlated to larger structural changes.

Overall, the limited proteolysis of the two different forms of TAU using the Dark-LiP methodology generated 412 peptides with significant difference in abundance.

Accordingly to the description above, peptides represented inside the **triangle** area in **Fig. 7** are high in the y-axis and low in the x-axis, thus have high -LogPvalue and low fold change. This means that the difference in the abundance of these peptides was low between monomer TAU and fibrillar TAU (which can be associated with mild structural change), but the quantification was very reproducible among the three replicates of the same condition (low standard deviation).

The peptide inside the **circle shape** in **Fig. 7** has a high value in the x-axis and low value in the y-axis (thus high fold change and relatively low -LogPvalue). Consequently, this peptide can be associated with a strong structural difference between the two variants of TAU, but the variability in the peptide abundance was high among measurements (leading to relatively low statistical significance).

Peptides inside the **rectangle** in **Fig. 7** have middle x-values and y-values, thus have a middle fold change and a middle -LogPvalue. These peptides can be used to identify structural changes between TAU variants in a robust way, since they can be measured reproducibly between replicate measurements, and the difference in abundance is also relatively high.

For our analysis, we considered monomeric TAU as the first variable, x₁ in equation 1, and fibrillar TAU as second variable, x₂ in equation 1. Since **Fig. 7** shows that the majority of the TAU peptides with high statistical significance and high fold change are located on the positive region of the x-axis, it means that x₁ is larger than x₂, and consequently the peptide abundance is higher in monomer TAU than in fibrillar TAU. This shows that monomer TAU was more accessible to proteolytic fragmentation by proteinase-K than fibrillar TAU, as expected (described above).

Overall, example 3 demonstrate that the Dark-LiP technology can be used to show the difference accessibility of several regions of the monomer and fibrillar TAU towards proteolysis, consequently highlighting the different structure of the two variants of TAU.

### LIST OF REFERENCE SIGNS

| | | | |
|---|---|---|---|
| 100 | native proteins | | Spectrometry |
| 101a | ligand | LiP | limited proteolysis |
| 101b | control (vehicle) samples | MRM | multiple reaction monitoring |
| 102 | limited digest step | MS | mass spectrometry |
| 103 | filtration | RT | retention time |
| 104a/b | unique peptide populations | SILAC | stable isotope labelling with |
| DDA | data dependent acquisition | | amino acids in cell culture |
| DIA | data independent acquisition | SRM | selected reaction monitoring |
| LC | liquid chromatography | SWATH | Sequential Windowed |
| LC-MS | Liquid chromatography coupled to Mass | | Acquisition of All Theoretical Fragment Ion Mass Spectra |

## Claims

1. Method for the detection of a conformational state of at least one protein, said at least one protein being contained in a complex mixture, from a biological probe, of further proteins and/or other biomolecules, preferably a complex cell extract mixture, wherein said at least one protein in said complex mixture has been subjected to a condition inducing a structural change in said at least one protein, comprising, if needed after an extraction and/or lysis step, the following sequence of steps:
1. Limited proteolysis of the complex mixture under a native condition in which the at least one protein is in the original conformational state to be detected leading to a first fragment sample; directly followed by
2. Removal of large peptides and proteins or other biomolecules from said first fragment sample to form an enriched fragment sample;
3. Analytical analysis of the enriched fragment sample by quantitative mass spectrometry for the determination of fragments characteristic of having been the result of the limited proteolysis of step 1. as well as remaining after the removal step 2. for the determination of the conformational state of said at least one protein
wherein in step 2. peptides, proteins and/or other biomolecules having a molar weight larger than 20 kDa are removed from the first fragment sample
wherein the method omits a full trypsinization step under denaturing conditions after the limited proteolysis step 1.

2. Method according to claim 1, wherein for the detection of the conformational state as such in parallel to steps 1.-3. the original complex mixture with said at least one protein and without being subjected to said condition inducing a structural change is subjected to steps 1. - 3. for the generation of an enriched fragment control sample, and wherein the determination of the conformational state of the at least one protein is based on a quantitative comparison of the analytical analysis of the enriched fragment sample with the analytical analysis of the enriched fragment control sample
or wherein for the detection of a change of the conformational state depending on different conditions in the complex mixture, a first and a second complex mixture is generated by subjecting them to the different conditions inducing a structural change in said at least one protein, by individually subjecting the two complex mixtures to steps 1.-3., and wherein the determination of the conformational change of the at least one protein is based on a comparison of the analytical analysis of the first enriched fragment sample with the analytical analysis of the second enriched fragment sample.

3. Method according to any of the preceding claims, wherein the condition inducing a structural change in said at least one protein in said complex mixture is selected from the group consisting of: temperature change; pressure change; ionic strength change; pH change; metabolic stimulant change; ligand addition, including drug/small molecule addition, metabolite addition, protein addition, peptide addition, lipid addition, DNA addition, RNA addition, disease/health state or status and genetic variations, including mutations, or a combination thereof; addition of a chaotrope; chemical modification, including post-translational modifications, in particular phosphorylations, disulphide bridge formation, ADP-ribosylation, ubiquitination, SUMOylation, acetylation, methylation, oxidation, glycosylation, or a combination thereof.

4. Method according to any of the preceding claims, wherein in step 2. peptides and proteins are removed in a filtration, separation or another enrichment step, including size filtering; chromatography including size exclusion, hydrophobic or anion exchange chromatography; physical removal including phase separation, absorption, precipitation; filtration, separation or enrichment based on hydrophilic/hydrophobic properties; filtration, separation or enrichment based on electric/magnetic field; or a combination thereof.

5. Method according to any of the preceding claims, wherein in step 2. peptides, proteins and/or other biomolecules having a molar weight larger than 15 kDa, most preferably having a molar weight larger than 10 kDa are removed from the first fragment sample.

6. Method according to any of the preceding claims, wherein step 3. includes, before actual analysis, a proteomics workflow, in particular involving denaturation, C18 cleanup, or a combination thereof.

7. Method according to any of the preceding claims, wherein in the step 1. a proteolytic system selected from the group consisting of protease K, Thermolysin, Subtilisin, Pepsin, Papain, α-Chymotrypsin, Elastase, and mixtures thereof is used.

8. Method according to any of the preceding claims, wherein in the step 1. the proteolytic system is used at a concentration, with respect to the total biomolecular content in the sample, given as the ratio of enzyme to biomolecular content, in the range of 1/50-1/10000, preferably in the range of 1/100-1/1000 by weight.

9. Method according to any of the preceding claims, wherein the step 1. is carried out over a time span of 1-60 minutes, preferably in the range of 2-30 minutes, or 2 - 10 minutes or 2 - 5 minutes, further preferably at a temperature in the range of 20-40°C.

10. Method according to any of the preceding claims, wherein for quantitative determination heavy labelled fragments characteristic of being the result of the limited proteolysis of step 1. as well as remaining after the removal step 2., are spiked into the original complex mixture and/or into the first fragment sample and/or into the enriched fragment sample.

11. Method according to any of the preceding claims, wherein for the analytical analysis in step 3. specific, quantitative mass spectrometry-based assays in the form of selected reaction monitoring (SRM) and/or data-independent acquisition of product ion spectra is used.

12. Method according to any of the preceding claims, wherein the complex mixture of further proteins and/or other biomolecules is a complex native biological matrix.

13. Method according to any of the preceding claims, wherein the at least one protein is a protein based exclusively on proteinogenic amino acids, or is based on proteinogenic amino acids and carries post-translational modifications.

14. Use of a method according to any of the preceding claims for the determination, in a hypothesis-free manner, of a conformation of said at least one protein, said at least one protein having undergone conformational changes after perturbation induced in the investigated complex mixture, or of a medically relevant conformation of the protein, for the determination of protein-based drugs, for the influence of drugs or other ligands on proteins, or for quality control of protein-based pharmaceutical preparations.

15. Method according to any of the claims 1- 13 further comprising peptide fragment enrichment techniques such as TAILS for the peptides generated by the step 1.

## Patentansprüche

1. Verfahren zur Erkennung eines Konformationszustands mindestens eines Proteins, wobei das mindestens eine Protein in einer komplexen Mischung aus einer biologischen Probe, weiteren Proteinen und/oder anderen Biomolekülen, vorzugsweise einer komplexen Zellextraktmischung, enthalten ist, wobei das mindestens eine Protein in der komplexen Mischung einer Bedingung ausgesetzt wurde, die eine Strukturänderung des mindestens einen Proteins induziert, umfassend, wenn erforderlich nach einem Extraktions- und/oder Lyse-Schritt, die folgende Schrittfolge:
1. Begrenzte Proteolyse der komplexen Mischung unter einer nativen Bedingung, wobei sich das mindestens eine zu erkennende Protein im ursprünglichen Konformationszustand befindet, was zu einer ersten Fragmentprobe führt; direkt gefolgt von
2. Entfernung großer Peptide und Proteine oder anderer Biomoleküle aus der ersten Fragmentprobe, um eine angereicherte Fragmentprobe zu bilden;
3. Analytische Untersuchung der angereicherten Fragmentprobe durch quantitative Massenspektrometrie zur Bestimmung von Fragmenten, die charakteristisch für das Ergebnis der begrenzten Proteolyse aus Schritt 1. sind und die nach dem Entfernungsschritt 2. zur Bestimmung des Konformationszustands des mindestens einen Proteins verbleiben
wobei in Schritt 2. Peptide, Proteine und/oder andere Biomoleküle mit einem Molekulargewicht größer als 20 kDa aus der ersten Fragmentprobe entfernt werden wobei das Verfahren auf einen vollständigen Trypsinisierungsschritt unter denaturierenden Bedingungen nach dem begrenzten Proteolyseschritt 1. verzichtet.

2. Verfahren nach Anspruch 1, wobei zur Erkennung des Konformationszustands als solcher parallel zu den Schritten 1. bis 3. die ursprüngliche komplexe Mischung mit dem mindestens einen Protein, ohne dass sie der Bedingung ausgesetzt wird, die eine Strukturänderung induziert, den Schritten 1. bis 3. zur Erzeugung einer angereicherten Fragment-Kontrollprobe ausgesetzt wird, und wobei die Bestimmung des Konformationszustands des mindestens einen Proteins auf einem quantitativen Vergleich der analytischen Untersuchung der angereicherten Fragmentprobe mit der analytischen Untersuchung der angereicherten Fragment-Kontrollprobe basiert
oder wobei zur Erkennung einer Änderung des Konformationszustands in Abhängigkeit von unterschiedlichen Bedingungen in der komplexen Mischung eine erste und eine zweite komplexe Mischung durch deren Aussetzen unterschiedlichen Bedingungen, die eine Strukturänderung in dem mindestens einen Protein induzieren durch individuelles Aussetzen der zwei komplexen Mischungen den Schritten 1. bis 3. erzeugt werden, und wobei die Bestimmung der Konformationsänderung des mindestens einen Proteins auf einem Vergleich der analytischen Untersuchung der ersten angereicherten Fragmentprobe mit der analytischen Untersuchung der zweiten angereicherten Fragmentprobe basiert.

3. Verfahren nach einem der vorhergehenden Ansprüche, wobei die Bedingung, die eine Strukturänderung in dem mindestens einen Protein in der komplexen Mischung induziert, ausgewählt ist aus der Gruppe bestehend aus: Temperaturänderung; Druckänderung; Änderung der Ionenstärke; pH-Änderung; Änderung des Stoffwechselstimulans; Ligandenaddition, einschließlich des Hinzufügens von Arzneimitteln/kleinen Molekülen, Metabolitenaddition, Proteinaddition, Peptidaddition, Lipidaddition, DNA-Addition, RNA-Addition, Krankheits-/Gesundheitszustand oder - status und genetische Varianten, einschließlich Mutationen, oder eine Kombination davon; Addition eines Chaotropen; chemische Modifikation, einschließlich posttranslationaler Modifikationen, insbesondere Phosphorylierung, Disulfidbrückenbildung, ADP-Ribosylierung, Ubiquitinierung, SUMOylierung, Acetylierung, Methylierung, Oxidation, Glykosylierung oder eine Kombination davon.

4. Verfahren nach einem der vorhergehenden Ansprüche, wobei in Schritt 2. Peptide und Proteine in einem Filtrations-, Trenn- oder einem anderen Anreicherungsschritt entfernt werden, einschließlich Größenfiltration; Chromatographie, einschließlich Größenausschluss-, hydrophober oder Anionenaustauschchromatographie; physikalische Entfernung, einschließlich Phasentrennung, Absorption, Ausfällung; Filtration, Trennung oder Anreicherung basierend auf hydrophilen/hydrophoben Eigenschaften; Filtration, Trennung oder Anreicherung basierend auf einem elektrischen/magnetischen Feld; oder einer Kombination davon.

5. Verfahren nach einem der vorhergehenden Ansprüche, wobei in Schritt 2. Peptide, Proteine und/oder andere Biomoleküle mit einem Molekulargewicht größer als 15 kDa, am meisten bevorzugt mit einem Molekulargewicht größer als 10 kDa, aus der ersten Fragmentprobe entfernt werden.

6. Verfahren nach einem der vorhergehenden Ansprüche, wobei Schritt 3. vor der eigentlichen Analyse einen Proteomik-Workflow einschließt, der insbesondere eine Denaturierung, eine C18-Reinigung oder eine Kombination davon beinhaltet.

7. Verfahren nach einem der vorhergehenden Ansprüche, wobei in dem Schritt 1. ein proteolytisches System, ausgewählt aus der Gruppe bestehend aus Protease K, Thermolysin, Subtilisin, Pepsin, Papain, α-Chymotrypsin, Elastase und Mischungen davon, verwendet wird.

8. Verfahren nach einem der vorhergehenden Ansprüche, wobei in dem Schritt 1. das proteolytische System in einer Konzentration verwendet wird, die, bezüglich des gesamten biomolekularen Gehalts in der Probe, als Verhältnis von Enzym zu biomolekularem Gehalt im Bereich von 1/50 bis 1/10.000, vorzugsweise im Bereich von 1/100 bis 1/1.000 bezogen auf das Gewicht, gegeben ist.

9. Verfahren nach einem der vorhergehenden Ansprüche, wobei der Schritt 1. über einen Zeitraum von 1 bis 60 Minuten, vorzugsweise im Bereich von 2 bis 30 Minuten, oder 2 bis 10 Minuten oder 2 bis 5 Minuten, weiter bevorzugt bei einer Temperatur im Bereich von 20 bis 40 °C durchgeführt wird.

10. Verfahren nach einem der vorhergehenden Ansprüche, wobei zur quantitativen Bestimmung schwere markierte Fragmente, die charakteristisch für das Ergebnis der begrenzten Proteolyse von Schritt 1. sind und auch nach dem Entfernungsschritt 2. verbleiben, in die ursprüngliche komplexe Mischung und/oder in die erste Fragmentprobe und/oder in die angereicherte Fragmentprobe dotiert werden.

11. Verfahren nach einem der vorhergehenden Ansprüche, wobei für die analytische Untersuchung in Schritt 3. spezifische, quantitative massenspektrometrische Assays in Form von Selected Reaction Monitoring (SRM) und/oder datenunabhängiger Erfassung von Produktionenspektren verwendet werden.

12. Verfahren nach einem der vorhergehenden Ansprüche, wobei die komplexe Mischung aus weiteren Proteinen und/oder anderen Biomolekülen eine komplexe native biologische Matrix ist.

13. Verfahren nach einem der vorhergehenden Ansprüche, wobei das mindestens eine Protein ein Protein ist, das ausschließlich auf proteinogenen Aminosäuren basiert, oder auf proteinogenen Aminosäuren basiert und posttranslationale Modifikationen trägt.

14. Verwendung eines Verfahrens nach einem der vorhergehenden Ansprüche zur hypothesenfreien Bestimmung einer Konformation des mindestens einen Proteins, wobei das mindestens eine Protein nach einer in der untersuchten komplexen Mischung induzierten Störung Konformationsänderungen durchlaufen hat, oder einer medizinisch relevanten Konformation des Proteins zur Bestimmung von proteinbasierten Arzneimitteln, zum Einfluss von Arzneimitteln oder anderen Liganden auf Proteine oder zur Qualitätskontrolle von proteinbasierten pharmazeutischen Präparaten.

15. Verfahren nach einem der Ansprüche 1 bis 13, ferner umfassend Peptidfragment-Anreicherungstechniken wie zum Beispiel TAILS für die durch Schritt 1. erzeugten Peptide.

## Revendications

1. Procédé de détection d'un état conformationnel d'au moins une protéine, ladite au moins une protéine étant contenue dans un mélange complexe, à partir d'une sonde biologique, de protéines supplémentaires et/ou d'autres biomolécules, de préférence un mélange d'extraits cellulaires complexe, dans lequel ladite au moins une protéine dans ledit mélange complexe a été soumise à une condition induisant un changement structurel dans ladite au moins une protéine, comprenant, si nécessaire après une étape d'extraction et/ou de lyse, la séquence d'étapes suivante :
1. la protéolyse limitée du mélange complexe dans une condition native dans laquelle l'au moins une protéine est dans l'état conformationnel d'origine à détecter, conduisant à un premier échantillon de fragments ; directement suivie par
2. l'élimination des peptides et protéines de grande taille ou d'autres biomolécules dudit premier échantillon de fragments pour former un échantillon de fragments enrichi ;
3. l'analyse analytique de l'échantillon de fragments enrichi par spectrométrie de masse quantitative, pour la détermination de fragments ayant pour caractéristique d'être le résultat de la protéolyse limitée de l'étape 1. ainsi que de subsister après l'étape 2. d'élimination pour la détermination de l'état conformationnel de ladite au moins une protéine,
dans lequel à l'étape 2., les peptides, protéines et/ou autres biomolécules ayant un poids molaire supérieur à 20 kDa sont éliminés du premier échantillon de fragments,
dans lequel le procédé omet une étape complète de trypsinisation en conditions dénaturantes après l'étape 1. de protéolyse limitée.

2. Procédé selon la revendication 1, dans lequel pour la détection de l'état conformationnel en tant que tel en parallèle aux étapes 1. à 3., le mélange complexe d'origine avec ladite au moins une protéine et sans être soumis à ladite condition induisant un changement structurel est soumis aux étapes 1. à 3. pour la génération d'un échantillon témoin de fragments enrichi, et dans lequel la détermination de l'état conformationnel de l'au moins une protéine est basée sur une comparaison quantitative de l'analyse analytique de l'échantillon de fragments enrichi avec l'analyse analytique de l'échantillon témoin de fragments enrichi,
ou dans lequel pour la détection d'un changement de l'état conformationnel dépendant de différentes conditions dans le mélange complexe, un premier et un deuxième mélange complexe sont générés en les soumettant aux différentes conditions induisant un changement structurel dans ladite au moins une protéine, en soumettant individuellement les deux mélanges complexes aux étapes 1. à 3., et dans lequel la détermination du changement conformationnel de l'au moins une protéine est basée sur une comparaison de l'analyse analytique du premier échantillon de fragments enrichi avec l'analyse analytique du deuxième échantillon de fragments enrichi.

3. Procédé selon l'une quelconque des revendications précédentes, dans lequel la condition induisant un changement structurel dans ladite au moins une protéine dans ledit mélange complexe est sélectionnée dans le groupe consistant en : changement de température ; changement de pression ; changement de force ionique ; changement de pH ; changement de stimulant métabolique ; ajout de ligand, incluant ajout de médicament ou de petite molécule, ajout de métabolite, ajout de protéine, ajout de peptide, ajout de lipide, ajout d'ADN, ajout d'ARN, état pathologique/de santé et variations génétiques, incluant mutations, ou une combinaison de ceux-ci ; ajout d'un chaotrope ; modification chimique, incluant des modifications post-traductionnelles, en particulier phosphorylations, formation de ponts disulfure, ADP-ribosylation, ubiquitination, SUMOylation, acétylation, méthylation, oxydation, glycosylation, ou une combinaison de celles-ci.

4. Procédé selon l'une quelconque des revendications précédentes, dans lequel à l'étape 2., les peptides et les protéines sont éliminés dans une étape de filtration, de séparation ou une autre étape d'enrichissement, incluant filtrage par taille ; chromatographie incluant exclusion par taille, chromatographie hydrophobe ou d'échange d'anions ; élimination physique incluant séparation de phase, absorption, précipitation ; filtration, séparation ou enrichissement sur la base de propriétés hydrophiles/hydrophobes ; filtration, séparation ou enrichissement sur la base d'un champ électrique/magnétique ; ou une combinaison de ceux-ci.

5. Procédé selon l'une quelconque des revendications précédentes, dans lequel à l'étape 2., les peptides, protéines et/ou autres biomolécules ayant un poids molaire supérieur à 15 kDa, ayant le plus préférablement un poids molaire supérieur à 10 kDa sont éliminés du premier échantillon de fragments.

6. Procédé selon l'une quelconque des revendications précédentes, dans lequel l'étape 3. inclut, avant l'analyse réelle, un flux de travail protéomique, impliquant en particulier une dénaturation, une purification sur C18, ou une combinaison de ceux-ci.

7. Procédé selon l'une quelconque des revendications précédentes, dans lequel à l'étape 1., un système protéolytique sélectionné dans le groupe consistant en protéase K, thermolysine, subtilisine, pepsine, papaïne, α-chymotrypsine, élastase et mélange de ceux-ci est utilisé.

8. Procédé selon l'une quelconque des revendications précédentes, dans lequel à l'étape 1., le système protéolytique est utilisé à une concentration, par rapport au contenu biomoléculaire total dans l'échantillon, exprimée comme le rapport entre l'enzyme et le contenu biomoléculaire, dans la plage de 1/50 à 1/10000, de préférence dans la plage de 1/100 à 1/1000 en poids.

9. Procédé selon l'une quelconque des revendications précédentes, dans lequel l'étape 1. est réalisée sur un intervalle de temps de 1 à 60 minutes, de préférence dans la plage de 2 à 30 minutes, ou de 2 à 10 minutes ou de 2 à 5 minutes, de préférence en outre à une température dans la plage de 20 à 40 °C.

10. Procédé selon l'une quelconque des revendications précédentes, dans lequel pour la détermination quantitative, des fragments marqués lourds ayant pour caractéristique d'être le résultat de la protéolyse limitée de l'étape 1., ainsi que de subsister après l'étape 2. d'élimination, sont ajoutés dans le mélange complexe d'origine et/ou dans le premier échantillon de fragments et/ou dans l'échantillon de fragments enrichi.

11. Procédé selon l'une quelconque des revendications précédentes, dans lequel pour l'analyse analytique de l'étape 3., des dosages quantitatifs spécifiques basés sur la spectrométrie de masse sous la forme d'une surveillance de réaction sélectionnée (SRM) et/ou d'une acquisition indépendante des données de spectres d'ions produits sont utilisés.

12. Procédé selon l'une quelconque des revendications précédentes, dans lequel le mélange complexe de protéines supplémentaires et/ou d'autres biomolécules est une matrice biologique native complexe.

13. Procédé selon l'une quelconque des revendications précédentes, dans lequel l'au moins une protéine est une protéine basée exclusivement sur des acides aminés protéinogènes, ou est basée sur des acides aminés protéinogènes et porte des modifications post-traductionnelles.

14. Utilisation d'un procédé selon l'une quelconque des revendications précédentes pour la détermination, sans hypothèse préalable, d'une conformation de ladite au moins une protéine, ladite au moins une protéine ayant subi des changements conformationnels après la perturbation induite dans le mélange complexe étudié, ou d'une conformation médicalement pertinente de la protéine, pour la détermination de médicaments à base de protéines, pour l'influence de médicaments ou d'autres ligands sur les protéines, ou pour le contrôle qualité de préparations pharmaceutiques à base de protéines.

15. Procédé selon l'une quelconque des revendications 1 à 13, comprenant en outre des techniques d'enrichissement de fragments de peptides telles que TAILS pour les peptides générés par l'étape 1.
